# EUROPÄISCHE PATENTSCHRIFT

(11) **EP 0 822 179 B1**
(45) Veröffentlichungstag und Bekanntmachung des Hinweises auf die Patenterteilung: **24.10.2001**
(21) Anmeldenummer: 97112341.9
(22) Anmeldetag: 18.07.1997
(51) Int. Cl.: C07C 209/60

(54) **Verfahren zur Herstellung von Aminen aus Olefinen an Zeolithen mit NES-Struktur**
Process for the preparation of amines from olefines using zeolites with nes-structure
Procédé pour la préparation d'amines à partir d'oléfines utilisant des zéolites à structure nes

(30) Priorität: 30.07.1996 DE 19630670
(43) Veröffentlichungstag der Anmeldung: 04.02.1998
(73) Patentinhaber: BASF AKTIENGESELLSCHAFT, 67056 Ludwigshafen (DE)
(72) Erfinder: Eller, Karsten, Dr., 67059 Ludwigshafen (DE); Kummer, Rudolf, Dr., 67227 Frankenthal (DE); Dernbach, Matthias, Dr., 69214 Eppelheim (DE)

(56) Entgegenhaltungen:
- EP-A- 0 378 916
- EP-A- 0 587 424
- EP-A- 0 778 259
- US-A- 5 254 514
- W. M. MEIER ET AL.: "Atlas of zeolite, structure Types, pages 162-163", 1996, ELSEVIER,

## Beschreibung

Die vorliegende Erfindung betrifft ein Verfahren zur Herstellung von Aminen durch Umsetzung von Ammoniak oder primären oder sekundären Aminen mit Olefinen bei erhöhten Temperaturen und Drükken in Gegenwart von Zeolithen mit NES-Struktur.

Eine Übersicht über die Methoden zur Aminierung von Olefinen wird in "Functionalisation of Alkenes: Catalytic Amination of Monoolefins", J.J. Brunet et al. J.Mol.Catal., 49 (1989), Seiten 235 bis 259 gegeben.

Grundsätzlich gibt es zwei Katalysemechanismen. Das Olefin wird über einen Metallkomplex koordiniert. Diese aktivierte Spezies kann von dem nucleophilen Amin angegriffen werden und ein höher aminiertes Produkt bilden. Das Amin kann an Säurezentren oder an Metallzentren (via Metallamide) chemisorbiert werden und so aktiviert mit dem Olefin umgesetzt werden.

Gut geeignete Katalysatoren sind Zeolithe. Sie zeichnen sich durch eine hohe Anzahl an katalytisch aktiven Zentren aus kombiniert mit einer großen Oberfläche. Die beschriebenen Zeolithe unterscheiden sich im Typ und in der Nachbehandlung (z.B. thermische Behandlung, Dealuminierung, Säurebehandlung, Metallioneneintausch, etc.). Beispiele hierfür finden sich in US-A-4 375 002, US-A-4 536 602, EP-A-305 564, EP-A-101 921, DE-A-42 06 992.

Aus EP-A-133 938, EP-A-431 451 und EP-A-132 736 sind Verfahren bekannt, bei denen Bor-, Gallium-, Alumino- und Eisensilikatzeolithe für die Herstellung von Aminen aus Olefinen verwendet werden und auf die Möglichkeit der Dotierung dieser Zeolithe mit Alkali-, Erdalkali- und Übergangsmetallen hingewiesen wird.

Aus CA-A-2 092 964 ist ein Verfahren zur Herstellung von Aminen aus Olefinen bekannt, bei dem BETA-Zeolithe, die als kristalline Aluminosilikate bestimmter Zusammensetzung mit einer Porengröße von mehr als 5 Å definiert sind, eingesetzt werden. Bevorzugt werden metall- oder halogenmodifizierte Beta-Zeolithe eingesetzt.

Alle Verfahren zur Synthese von Aminen aus Olefinen an diesen Katalysatoren zeichnen sich durch eine geringe Amin-Ausbeute oder geringe Raum-Zeit-Ausbeute aus, oder führen zu einer raschen Desaktivierung der Katalysatoren.

Der vorliegenden Erfindung lag daher die Aufgabe zugrunde, diesen Nachteilen abzuhelfen.

Demgemäß wurde ein neues und verbessertes Verfahren zur Herstellung von Aminen der allgemeinen Formel I 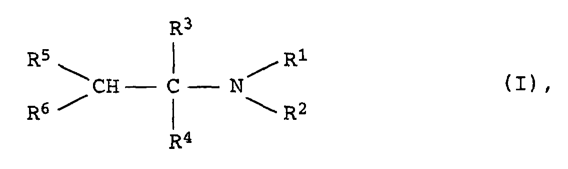 in der
- R¹,R²,R³,R⁴,R⁵,R⁶: Wasserstoff, C₁- bis C₈-Alkyl, C₅- bis C₈-Cyclo-alkyl, C₄- bis C₁₂-Alkyl-cycloalkyl oder C₄- bis C₁₂-Cycloalkyl-alkyl,
- R¹ und R²: gemeinsam -(CH₂)₄-, -(CH₂)₅- oder -(CH₂)₇-
- R³ oder R⁵: C₂₁- bis C₂₀₀-Alkyl, C₂₁- bis C₂₀₀-Alkenyl oder gemeinsam eine C₃- bis C₈-Alkylendikette
bedeuten, durch Umsetzung von Olefinen der allgemeinen Formel II 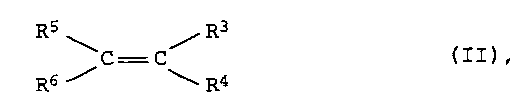 in der R³, R⁴, R⁵ und R⁶ die oben genannten Bedeutungen haben, mit Ammoniak oder primären oder sekundären Aminen der allgemeinen Formel III 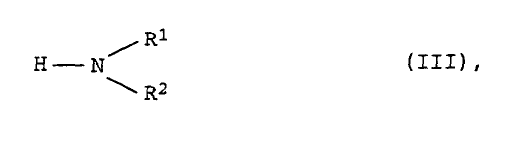 in der R¹ und R² die oben genannten Bedeutungen haben, bei Temperaturen von 200 bis 350°C und Drücken von 100 bis 300 bar in Gegenwart eines Heterogenkatalysators gefunden, welches dadurch gekennzeichnet ist, daß man als Heterogenkatalysator Zeolithe mit NES-Struktur einsetzt, mit der Maßgabe, daß der Zeolith SSZ-37 ausgenommen ist.

Das erfindungsgemäße Verfahren kann wie folgt durchgeführt werden:
Das Olefin II und Ammoniak oder das primäre oder sekundären Amin III kann bei Temperaturen von 200 bis 350°C, bevorzugt 220 bis 330°C, besonders bevorzugt 230 bis 320°C und Drücken von 100 bis 300 bar, bevorzugt 120 bis 300 bar, besonders bevorzugt 140 bis 290 bar in Gegenwart von Zeolithen mit NES-Struktur als Katalysator z.B. in einem Druck-Reaktor umsetzt werden, und bevorzugt das erhaltene Amin abgetrennt und die nichtumgesetzten Einsatzstoffe zurückführt werden.

Das vorliegende Verfahren zeichnet sich durch eine sehr gute Ausbeute bei hoher Selektivität und bei hoher Raum-Zeit-Ausbeute aus. Zudem ist die Desaktivierung des Katalysators zurückgedrängt worden.

Das erfindungsgemäße Verfahren zeichnet sich dadurch aus, daß bereits mit niedrigem Ammoniak- bzw. Amin- Überschuß eine hohe Selektivität an gewünschtem Reaktionsprodukt erzielt und die Dimerisierung und/oder Oligomerisierung des eingesetzten Olefins vermieden wird.

Eine Ausführungsform dieses Verfahrens besteht darin, daß man Ammoniak und/oder Amine III zusammen mit dem Olefin II im molaren Verhältnis von 1:1 bis 5:1 gemischt einem Festbettreaktor zuführt und bei einem Druck von 100 bis 300 bar und einer Temperatur von 200 bis 350°C in der Gasphase oder im überkritischen Zustand umsetzt.

Aus dem Reaktionsaustrag kann das gewünschte Produkt mit Hilfe bekannter Methoden, beispielsweise Destillation oder Extraktion, erhalten und nötigenfalls mittels weiterer Trennoperationen auf die gewünschte Reinheit gebracht werden. Die nichtumgesetzten Eingangsstoffe werden in der Regel bevorzugt in den Reaktor zurückgeführt.

Man kann einfach oder mehrfach ungesättigte Olefine II, insbesondere solche mit 2 bis 10 C-Atomen bzw. deren Mischungen und Polyolefine als Ausgangsstoffe verwenden. Wegen der geringer ausgeprägten Polymerisationsneigung eignen sich Monoolefine besser als Di- und Polyolefine, doch können diese mit Hilfe höherer Ammoniak- bzw. Aminüberschüsse ebenso selektiv umgesetzt werden. Die Lage des Gleichgewichts und damit der Umsatz zum gewünschten Amin ist sehr stark vom gewählten Reaktionsdruck abhängig. Hoher Druck begünstig das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Zudem ist eine Temperaturerhöhung aus thermodynamischer Sicht nicht vorteilhaft. Die Lage des Temperaturoptimums bezüglich Umsatz und Selektivität ist von der Konstitution des Olefins, des eingesetzten Amins und des Katalysators abhängig und liegt meist im Bereich von 200 bis 350°C.

Als Katalysatoren für die Aminierung von Olefinen eignen sich Zeolithe mit NES-Struktur, bevorzugt NU-87 Zeolithe, die beispielsweise aus EP-A-377 291 bekannt sind.

Zeolithe mit NES-Struktur besitzen ein zweidimensionales Porensystem mit den ungefähren Abmessungen 4.7 x 6.0 Å (Meier, Olson, Atlas of Zeolite Structure Types, 3rd Ed., 1992, Butterworth-Heinemann, Seite 154 bis 155). NES-Struktur besitzt beispielsweise der NU-87 (Shannon et al., Nature 353 (1991) S. 417 bis 420). Die Struktur von SSZ-37 (US 5 254 514) ist noch nicht endgültig geklärt, sie scheint aber verwandt zu NU-87 zu sein (Nakagawa, Stud. Surf. Sci. Catal. 84 (1994) Seite 323 bis 330), so daß er im Sinne dieser Anmeldung auch unter die erfindungsgemäßen Zeolithe mit NES-Struktur fallen soll.

Neben den NES-Zeolithen mit Aluminium als dreiwertigem Element in der SiO₂-Matrix, wie es z.B. beim NU-87 realisiert ist, sind im Sinne dieser Anmeldung weiterhin andere Elemente möglich, wenn durch ihren Einbau acide Zentren geschaffen werden. Dies ist beispielsweise bei Borzeolithen, Eisenzeolithen oder Galliumzeolithen der Fall. Das molare Verhältnis von SiO₂ zu den Oxiden der dreiwertigen Elemente, das sogenannte Modul SiO₂/M₂O₃ (M = Al, B, Ga, Fe), kann je nach Zeolithklasse zwischen nahezu unendlich bis zu einigen Zehn variieren.

Neben den klassischen Zeolithen auf SiO₂-Basis können analoge Strukturen auch auf Basis von Aluminiumphosphaten realisiert werden, den sogenannten AlPOs. Enthalten diese Aluminium und Phosphor im Verhältnis größer 1, so sind sie ebenfalls sauer und können im Sinne der Erfindung eingesetzt werden. Ist ein Teil des Phosphors und/oder gleichzeitig Aluminium und Phosphor durch Silizium ersetzt, so erhält man die sogenannten SAPOs, die ebenfalls sauer sind. Kommen neben Aluminium und Phosphor noch verschiedene Metallionen wie beispielsweise Li, B, Be, Mg, Ti, Mn, Fe, Co, Zn, Ga, Ge, As vor, so spricht man von MeAPOs, oder bei gleichzeitiger Anwesenheit von Silizium von MeAPSOs, bei denen die negative Ladung des MeₐAl_{b}P_{c}Si_{d}Oₑ-Gerüstes jeweils durch Kationen kompensiert wird. Alle derartigen Molekularsiebe mit NES-Struktur fallen unter die erfindungsgemäßen Katalysatoren.

Die erfindungsgemäßen Zeolithe mit NES-Struktur können als solche verformt werden, oder aber auch mit einem Bindemittel im Verhältnis 98:2 bis 40:60 Gew.-% zu Strängen oder Tabletten. Als Bindemittel eignen sich verschiedene Aluminiumoxide, bevorzugt Boehmit, amorphe Aluminosilikate mit einem SiO₂/Al₂O₃-Verhältnis von 25:75 bis 95:5, Siliciumdioxid, bevorzugt hochdisperses SiO₂, Gemische aus hochdispersem SiO₂ und hochdispersem Al₂O₃, hochdisperses TiO₂ sowie Tone. Nach der Verformung werden die Extrudate oder Preßlinge zweckmäßig bei 110°C/16 Stunden getrocknet und bei 200 bis 500°C/2 bis 16 Stunden calciniert, wobei die Calcinierung auch direkt im Aminierungsreaktor erfolgen kann.

Zur Erhöhung der Selektivität, der Standzeit und der Anzahl der möglichen Regenerierungen kann man verschiedene Modifizierungen an den erfindungsgemäßen Zeolithkatalysatoren mit NES-Struktur vornehmen.

Eine Modifizierung der Katalysatoren besteht darin, daß man die unverformten oder die verformten Zeolithe mit Alkalimetallen wie Na und K, Erdalkalimetallen wie Ca, Mg, Erdmetallen wie Tl, Übergangsmetallen wie z.B. Ti, Zr, Mn, Fe, Mo, Cu, Zn, Cr, Edelmetallen und/oder seltenen Erdmetallen wie z.B. La, Ce oder Y ionenaustauschen bzw. dotieren kann.

Eine vorteilhafte Ausführungsform besteht darin, daß man die verformten erfindungsgemäßen Zeolithe mit NES-Struktur in einem Strömungsrohr vorlegt und bei 20 bis 100°C z.B. ein Halogenid ein Acetat, ein Oxalat, ein Citrat oder ein Nitrat der oben beschriebenen Metalle in gelöster Form darüberleitet. Ein derartiger Ionenaustausch kann z.B. an der Wasserstoff-, Ammonium-und Alkaliform der erfindungsgemäßen Zeolithe mit NES-Struktur vorgenommen werden.

Eine weitere Möglichkeit der Metallaufbringung auf die erfindungsgemäßen Zeolithe mit NES-Struktur besteht darin, daß man das Material z.B. mit einem Halogenid, einem Acetat, einem Oxalat, einem Citrat, einem Nitrat oder einem Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten Zeolithen mit NES-Struktur kann eine Nachbehandlung mit Wasserstoff und/oder mit Wasserdampf günstig sein.

Eine weitere Möglichkeit der Modifizierung besteht darin, daß man die erfindungsgemäßen Zeolithe mit NES-Struktur - verformt oder unverformt - einer Behandlung mit Säuren, wie Salzsäure (HCl), Flußsäure (HF), Schwefelsäure (H₂SO₄), Phosphorsäure (H₃PO₄), Oxalsäure (HO₂C-CO₂M) oder deren Gemischen unterwirft.

Eine besondere Ausführungsform besteht darin, daß man die erfindungsgemäßen Zeolithe mit NES-Struktur vor der Verformung mit einer der genannten Säuren 0,001 n bis 2 n, bevorzugt 0,05 bis 0,5 n 1 bis 100 Stunden unter Rückfluß behandelt. Nach Abfiltrieren und Auswaschen wird in der Regel bei 100 bis 160°C getrocknet und bei 200 bis 600°C calciniert. Eine weitere besondere Ausführungsform liegt in einer Säure-Behandlung der erfindungsgemäßen Zeolithe mit NES-Struktur nach ihrer Verformung mit Bindemittel. Hierbei wird der erfindungsgemäße Zeolith in der Regel 1 bis 3 Stunden zwischen 60 und 80°C mit einer 3 bis 25%igen, insbesondere mit einer 12 bis 20%igen Säure behandelt, anschließend ausgewaschen, bei 100 bis 160°C getrocknet und bei 200 bis 600°C calciniert. Auch hier kann die Calcinierung wieder direkt im Aminierungsreaktor erfolgen.

Eine andere Möglichkeit der Modifizierung ist gegeben durch einen Austausch mit Ammonsalzen, z.B. mit NH₄Cl oder mit Mono-, Di- oder Polyaminen. Hierbei wird der mit Bindemittel verformte Zeolith in der Regel bei 60 bis 80°C mit 10 bis 25%iger, bevorzugt 20%iger NH₄Cl-Lösung 2 h kontinuierlich in gewichtsmäßiger Zeolith/Ammonchlorid-Lösung von 1:15 ausgetauscht und danach bei 100 bis 120°C getrocknet.

Eine weitere Modifikation, die an den erfindungsgemäßen Zeolithen vorgenommen werden kann, ist die Dealuminierung im Falle von Aluminium-Zeolithen, bei der ein Teil der Aluminiumatome durch Silicium ersetzt wird oder die Zeolithe durch beispielsweise hydrothermale Behandlung in ihrem Aluminiumgehalt abgereichert werden. An eine hydrothermale Dealuminierung schließt sich vorteilhafterweise eine Extraktion mit Säuren oder Komplexbildnern an, um gebildetetes Nichtgitteraluminium zu entfernen. Der Ersatz von Aluminium durch Silicium kann beispielsweise mit Hilfe von (NH₄)₂SiF₆ oder SiCl₄ erfolgen. Beispiele für Dealuminierungen von Y-Zeolithen finden sich in Corma et al., Stud. Surf. Sci. Catal. 37 (1987), Seiten 495 bis 503. Bei anderen dreiwertigen Oxiden kann entsprechend das Modul vergrößert werden, indem ein Teil des Bors, des Eisens oder des Galliums herausgelöst oder durch Silicium ersetzt wird.

Die Katalysatoren kann man als Stränge mit Durchmessern von z.B. 1 bis 4 mm oder als Tabletten mit z.B. 3 bis 5 mm Durchmesser für die Aminierung der Olefine einsetzen.

Aus dem beispielsweise zu Strängen verformten Katalysator kann man durch Mahlen und Sieben ein Wirbelgut in der Größe von 0,1 bis 0,8 mm erhalten.

Die Substituenten R¹, R², R³, R⁴, R⁵ und R⁶ in den Verbindungen I, II und III haben folgende Bedeutungen:
R¹,R²,R³,R⁴,R⁵,R⁶
- Wasserstoff,
- C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
- C₅- bis C₈-Cycloalkyl wie Cyclopentyl, Cyclohexyl, Cycloheptyl und Cyclooctyl,
- C₄- bis C₁₂-Alkyl-cycloalkyl, besonders bevorzugt C₅- bis C₁₀-Alkyl-cycloalkyl,
- C₄- bis C₁₂-Cycloalkyl-alkyl, besonders bevorzugt C₅- bis C₁₀-Cycloalkyl-alkyl,
R¹ und R²
- gemeinsam -(CH₂)₄-, -(CH₂)₅- oder -(CH₂)₇-,
   R³ und R⁵
- gemeinsam eine C₃- bis C₈-Alkylendikette, besonders bevorzugt -(CH₂)₃-, -(CH₂)₄-, -(CH₂)₅-, -(CH₂)₆- und -(CH₂)₇-, insbesondere -(CH₂)₃- und -(CH₂)₄-.

### Beispiele

### Katalysatorsynthesen

### Katalysator A: Na-NU-87

Lösung A wurde aus 11.4 g NaOH und 6.6 g Natriumaluminat in 360 g Wasser angesetzt. Lösung B wurde aus 56.4 g Decamethoniumbromid in 360 g Wasser angesetzt. Lösung C wurde aus 165 g Ludox© AS 40 in 55.2 g Wasser angesetzt. Lösung A wurde unter Rühren zu Lösung C gegeben, noch 5 min weitergerührt und dann Lösung B hinzugefügt. Weitere 255 g Wasser wurden zugegeben und weitere 30 min gerührt. Die Mischung wurde dann in einen Autoklaven überführt und 400 h bei 180°C unter dem Eigendruck gerührt kristallisiert. Der gebildete Zeolith wurde abfiltriert und gewaschen, 4 h bei 110°C getrocknet und 16 h bei 500°C kalziniert. Er besaß ein Modul von 30.

### Katalysator B: H-NU-87

50 g Katalysator A wurden mit 750 g einer 20 %iger NH₄Cl-Lösung bei 80°C für 2 h gerührt und anschließend abfiltriert und mit 2 l Wasser gewaschen. Nach erneutem NH₄Cl-Austausch und Nachwaschen wurde der Zeolith 2 h bei 120°C getrocknet und 5 h bei 500°C kalziniert. Der gesamte Vorgang wurde dann noch einmal wiederholt.

45 g des ausgetauschten Zeolithen wurden mit 30 g Boehmit und 1.5 g Ameisensäure, im Kneter kompaktiert und unter Wasserzusatz (50 ml) 40 min verknetet. In einer Strangpresse wurden mit einem Pressdruck von 50 bar 2 mm Stränge erzeugt und diese 16 h bei 120°C getrocknet und 16 h bei 500°C kalziniert. Sie besaßen einen Rest-Natrium-Gehalt von 0.13 % und 402 m²g⁻¹ BET-Oberfläche.

### Katalysator C: H-NU-87

20 g Katalysator B wurden in einem Strömungsrohr mit 20 %iger NH₄NO₃-Lösung bei 80°C für 6 h ionengetauscht. Nach Waschung mit 10 l Wasser wurde für 4 h bei 120°C getrocknet und 5 h bei 500°C kalziniert. Die Stränge enthielten nach der Kalzinierung noch 0.05 % Na.

### Katalysator D: Na-NU-87

Katalysator D wurde analog zu Katalysator A hergestellt, aber nur 300 h kristallisiert.

### Katalysator E: H-NU-87

Katalysator E wurde analog zu Katalysator B ionenausgetauscht, aber aus Katalysator D. Der Restnatriumgehalt betrug weniger als 0.01 %. 45 g des ausgetauschten Zeolithen wurden mit 30 g Boehmit und 1.5 g Ameisensäure, im Kneter kompaktiert und unter Wasserzusatz (64 ml) 35 min verknetet. In einer Strangpresse wurden mit einem Pressdruck von 55 bar 2 mm Stränge erzeugt und diese 4 h bei 120°C getrocknet und 16 h bei 500°C kalziniert.

### Aminierungsbeispiele

Die Versuche wurden in einem Rohrreaktor (6 mm Innendurchmesser) unter isothermen Bedingungen bei 260°C bis 300C und einem Druck von 280 bar mit einem Gemisch aus Isobuten und Ammoniak im molaren Verhältnis von 1:1,5 durchgeführt. Die Reaktionsprodukte wurden im Gaschromatographen analysiert.

Die Ergebnisse sind in Tabelle 1 zusammengestellt.

**Tabelle 1**

| tert.-Butylamin (NH₃: C₄H₈ = 1,5) | | | | | | |
|---|---|---|---|---|---|---|
| Kat. | Temperatur [°C] | tert.-Butylamin-Ausbeute [Gew.-%] | | | | Litergewicht |
| | | WHSV 0,38 [g/g·h] | WHSV 0,75 [g/g·h] | WHSV 1,5 [g/g·h] | WHSV 3 [g/g·h] | [kg/l] |
| B | 260 | 25,10 | 22,56 | 19,36 | 14,43 | 0,56 |
| B | 270 | | 21,91 | 20,11 | 17,94 | 0,56 |
| B | 280 | | 18,06 | 17,69 | 17,13 | 0,56 |
| B | 300 | | | | 12,73 | 0,56 |
| C | 270 | | 21,45 | 19,13 | 16,18 | 0,56 |
| E | 270 | | 21,00 | 19,89 | 16,78 | 0,45 |

## Patentansprüche

1. Verfahren zur Herstellung von Aminen der allgemeinen Formel I 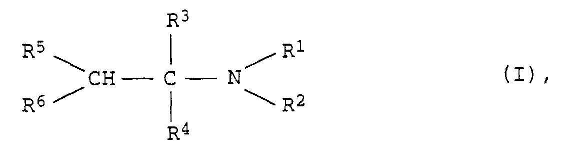 in der
R¹,R²,R³,R⁴,R⁵,R⁶ Wasserstoff, C₁- bis C₈-Alkyl, C₅- bis C₈-Cycloalkyl, C₄- bis C₁₂-Alkyl-cycloalkyl oder C₄- bis C₁₂-Cycloalkyl-alkyl,
R¹ und R² gemeinsam -(CH₂)₄-, - (CH₂)₅- oder -(CH₂)₇- R³ und R⁵ gemeinsam eine C₃- bis C₈-Alkylendikette
bedeuten, durch Umsetzung von Olefinen der allgemeinen Formel II 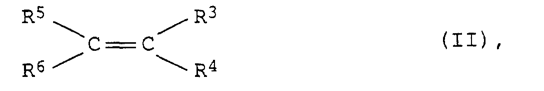 in der R³, R⁴, R⁵ und R⁶ die oben genannten Bedeutungen haben, mit Ammoniak oder primären oder sekundären Aminen der allgemeinen Formel III 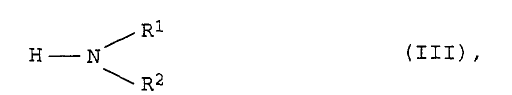 in der R¹ und R² die oben genannten Bedeutungen haben, bei Temperaturen von 200 bis 350°C und Drücken von 100 bis 300 bar in Gegenwart eines Heterogenkatalysators, **dadurch gekennzeichnet, daß** man als Heterogenkatalysator Zeolithe mit NES-Struktur einsetzt, mit der Maßgabe, daß der Zeolith SSZ-37 ausgenommen ist.

2. Verfahren zur Herstellung von Aminen I nach Anspruch 1, **dadurch gekennzeichnet, daß** man das gebildete Amin I abtrennt und die nichtumgesetzten Einsatzstoffe II und III zurückführt.

3. Verfahren zur Herstellung von Aminen nach den Ansprüchen 1 bis 2, **dadurch gekennzeichnet, daß** man als Olefin II Isobuten, Diisobuten, Cyclopenten, Cyclohexen oder Polyisobuten einsetzt.

4. Verfahren zur Herstellung von Aminen nach den Ansprüchen 1 bis 3, **dadurch gekennzeichnet, daß** man als Heterogenkatalysatoren Zeolithe mit NES-Struktur in der H-Form einsetzt.

5. Verfahren zur Herstellung von Aminen nach den Ansprüchen 1 bis 4, **dadurch gekennzeichnet, daß** man als Heterogenkatalysatoren Zeolithe mit NES-Struktur, die mit einer Säure, insbesondere einer aus der Gruppe Salzsäure, Flußsäure, Schwefelsäure, Phosphorsäure, Oxalsäure oder deren Gemischen, behandelt sind, einsetzt.

6. Verfahren zur Herstellung von Aminen nach den Ansprüchen 1 bis 5, **dadurch gekennzeichnet, daß** man als Heterogenkatalysatoren Zeolithe mit NES-Struktur, die mit einem oder mehreren Übergangsmetallen dotiert sind, einsetzt.

7. Verfahren zur Herstellung von Aminen nach den Ansprüchen 1 bis 6, **dadurch gekennzeichnet, daß** man als Heterogenkatalysatoren Zeolithe mit NES-Struktur, die mit einem oder mehreren Elementen der Seltenen Erden dotiert sind, einsetzt.

8. Verfahren zur Herstellung von Aminen nach den Ansprüchen 1 bis 7, **dadurch gekennzeichnet, daß** man als Heterogenkatalysatoren Zeolithe mit NES-Struktur in der Ammoniumform einsetzt.

9. Verfahren zur Herstellung von Aminen nach den Ansprüchen 1 bis 8, **dadurch gekennzeichnet, daß** man als Heterogenkatalysatoren Zeolithe mit NES-Struktur einsetzt, die mit einem oder mehreren Elementen aus der Gruppe der Alkali-, Erdalkalimetalle oder Erdmetalle dotiert sind.

10. Verfahren zur Herstellung von Aminen nach den Ansprüchen 1 bis 9, **dadurch gekennzeichnet, daß** man als Heterogenkatalysatoren Zeolithe mit NES-Struktur einsetzt, die mit einem Bindemittel verformt und bei Temperaturen von 200 bis 600°C calciniert sind.

11. Verfahren zur Herstellung von Aminen nach den Ansprüchen 1 bis 10, **dadurch gekennzeichnet, daß** man als Heterogenkatalysatoren dealuminierte oder deborierte Zeolithe mit NES-Struktur einsetzt.

12. Verfahren zur Herstellung von Aminen nach den Ansprüchen 1 bis 11, **dadurch gekennzeichnet, daß** man als Heterogenkatalysatoren aus der Gruppe der Zeolithe mit NES-Struktur NU-87 Zeolithe einsetzt.

## Claims

1. A process for preparing amines of the general formula I 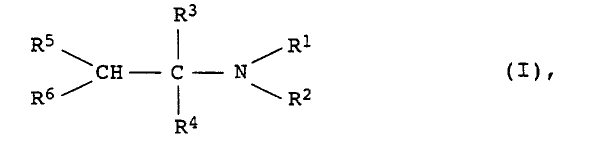 where
R¹,R²,R³,R⁴,R⁵,R⁶ are hydrogen, C₁-C₈-alkyl, C₅-C₈-cycloalkyl, C₄-C₁₂-alkyl-cycloalkyl or C₄-C₁₂-cycloalkyl-alkyl,
R¹ and R² are together -(CH₂)₄-, -(CH₂)₅- or -(CH₂)₇-and
R³ and R⁵ are together a divalent C₃-C₈-alkylene chain,
by reacting olefins of the general formula II 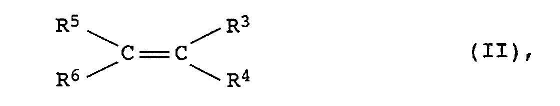 where R³, R⁴, R⁵ and R⁶ are as defined above, with ammonia or primary or secondary amines of the general formula III 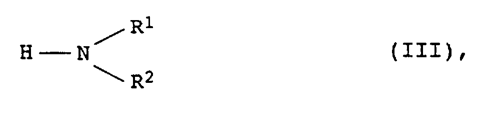 where R¹ and R² are as defined above, at from 200 to 350°C and pressures of from 100 to 300 bar in the presence of a heterogeneous catalyst, wherein the heterogeneous catalyst used is a zeolite having an NES structure, with the proviso that the zeolite SSZ-37 is excluded.

2. A process for preparing amines I as claimed in claim 1, wherein the amine I formed is separated off and the unreacted starting materials II and III are recirculated.

3. A process for preparing amines as claimed in claim 1 or 2, wherein the olefin II used is isobutene, diisobutene, cyclopentene, cyclohexene or polyisobutene.

4. A process for preparing amines as claimed in any of claims 1 to 3, wherein the heterogeneous catalyst used is a zeolite having an NES structure in the H form.

5. A process for preparing amines as claimed in any of claims 1 to 4, wherein the heterogeneous catalyst used is a zeolite having an NES structure which has been treated with an acid, in particular one selected from the group consisting of hydrochloric acid, hydrofluoric acid, sulfuric acid, phosphoric acid, oxalic acid and mixtures thereof.

6. A process for preparing amines as claimed in any of claims 1 to 5, wherein the heterogeneous catalyst used is a zeolite having an NES structure which is doped with one or more transition metals.

7. A process for preparing amines as claimed in any of claims 1 to 6, wherein the heterogeneous catalyst used is a zeolite having an NES structure which is doped with one or more elements of the rare earths.

8. A process for preparing amines as claimed in any of claims 1 to 7, wherein the heterogeneous catalyst used is a zeolite having an NES structure in the ammonium form.

9. A process for preparing amines as claimed in any of claims 1 to 8, wherein the heterogeneous catalyst used is a zeolite having an NES structure which is doped with one or more elements selected from the group consisting of the alkali metals, alkaline earth metals and earth metals.

10. A process for preparing amines as claimed in any of claims 1 to 9, wherein the heterogeneous catalyst used is a zeolite having an NES structure which has been shaped together with a binder and calcined at from 200 to 600°C.

11. A process for preparing amines as claimed in any of claims 1 to 10, wherein the heterogeneous catalyst used is a dealuminated or deborated zeolite having an NES structure.

12. A process for preparing amines as claimed in any of claims 1 to 11, wherein the heterogeneous catalyst selected from the group consisting of zeolites having an NES structure is an NU-87 zeolite.

## Revendications

1. Procédé pour la préparation d'amines répondant à la formule générale I 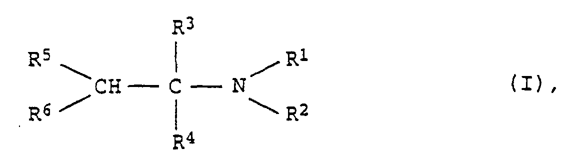 dans laquelle
R¹, R², R³, R⁴, R⁵, R⁶ représentent l'hydrogène, des groupes alkyle en C1-C8, cycloalkyle en C5-C8, alkyl-cycloalkyle en C4-C12 ou cycloalkyl-alkyle en C4-C12,
R¹ et R² forment ensemble -(CH₂)₄-, -(CH₂)₅- ou -(CH₂)₇-,
R³ et R⁵ forment ensemble une chaîne divalente alkylène en C3-C8,
par réaction d'oléfines de formule générale II 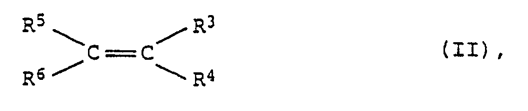 dans laquelle R³, R⁴, R⁵ et R⁶ ont les significations indiquées ci-dessus, avec l'ammoniac ou des amines primaires ou secondaires de formule générale III 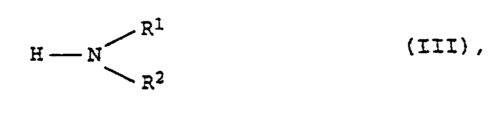 dans laquelle R¹ et R² ont les significations indiquées ci-dessus, à des températures de 200 à 350°C et des pressions de 100 à 300 bar en présence d'un catalyseur hétérogène, **caractérisé par le fait que** l'on utilise en tant que catalyseur hétérogène une zéolithe à structure NES sous réserve que la zéolithe SSZ-37 est exclue.

2. Procédé pour la préparation des amines I selon la revendication 1, **caractérisé par le fait que** l'on sépare l'amine I formée et on recycle les composants de départ II et III non convertis.

3. Procédé pour la préparation d'amines selon les revendications 1 et 2, **caractérisé par le fait que** l'oléfine II mise en oeuvre consiste en isobutène, diisobutène, cyclopentène, cyclohexène ou polyisobutène.

4. Procédé pour la préparation d'amines selon les revendications 1 à 3, **caractérisé par le fait que** l'on utilise en tant que catalyseur hétérogène une zéolithe à structure NES sous la forme H.

5. Procédé pour la préparation d'amines selon les revendications 1 à 4, **caractérisé par le fait que** l'on utilise, en tant que catalyseur hétérogène, une zéolithe à structure NES qui a été traitée par un acide, en particulier un acide du groupe de l'acide chlorhydrique, de l'acide fluorhydrique, de l'acide sulfurique, de l'acide phosphorique, de l'acide oxalique ou leurs mélanges.

6. Procédé pour la préparation d'amines selon les revendications 1 à 5, **caractérisé par le fait que** l'on utilise en tant que catalyseur hétérogène une zéolithe à structure NES dopée par un ou plusieurs métaux de transition.

7. Procédé pour la préparation d'amines selon les revendications 1 à 6, **caractérisé par le fait que** l'on utilise en tant que catalyseur hétérogène une zéolithe à structure NES qui a été dopée par un ou plusieurs éléments des terres rares.

8. Procédé pour la préparation d'amines selon les revendications 1 à 7, **caractérisé par le fait que** l'on utilise en tant que catalyseur hétérogène une zéolithe à structure NES sous la forme ammonium.

9. Procédé pour la préparation d'amines selon les revendications 1 à 8, **caractérisé par le fait que** l'on utilise, en tant que catalyseur hétérogène, une zéolithe à structure NES dopée par un ou plusieurs éléments du groupe des métaux alcalins, des métaux alcalino-terreux ou des métaux terreux.

10. Procédé pour la préparation d'amines selon les revendications 1 à 9, **caractérisé par le fait que** l'on utilise, en tant que catalyseur hétérogène, une zéolithe à structure NES qui a été moulée avec un liant et calcinée à des températures de 200 à 600°C.

11. Procédé pour la préparation d'amines selon les revendications 1 à 10, **caractérisé par le fait que** l'on utilise, en tant que catalyseur hétérogène, une zéolithe à structure NES désaluminisée ou déborée.

12. Procédé pour la préparation d'amines selon les revendications 1 à 11, **caractérisé par le fait que** l'on utilise, en tant que catalyseur hétérogène, du groupe des zéolithes à structures NES une zéolithe NU-87.
